# EUROPEAN PATENT APPLICATION

(11) **EP 2 230 240 A1**
(43) Date of publication of application: **22.09.2010**
(21) Application number: 08860779.1
(22) Date of filing: 11.12.2008
(51) Int. Cl.: C07D 477/00, A61K 31/427, A61P 31/04, C07D 487/04

(54) **STABLE CRYSTAL OF -LACTAM COMPOUND**

(30) Priority: 12.12.2007 JP 2007320687
(71) Applicant: Dainippon Sumitomo Pharma Co., Ltd., Osaka 541-8524 (JP)
(72) Inventor: YOKOYAMA, Tatsuo, Osaka-shi Osaka 541-8524 (JP); ITOH, Masanori, Osaka-shi Osaka 554-0022 (JP); TAKAMOTO, Koji, Osaka-shi Osaka 553-0001 (JP)
(74) Representative: Berryman, Natalia Grace
(86) International application number: PCT/JP2008/072504
(87) International publication number: WO 2009/075323

(57) **Abstract**

A stable crystal (Form I crystal) of (4R,5S,6S)-6-[(1R)-1-hydroxyethyl]-4-methyl-3-({4-[(5S)-5-methyl-2,5-dihydro-1H-pyrrol-3-yl]-1,3-thiazol-2-yl}thro)-7-oxo-1-azabicyclo[3.2.0]hept-2-ene-2-carboxylic acid which has preferable properties and a process for preparation Form I crystal which is **characterized** of desolvating a solvated crystal of its compound.

## Description

### TECHNICAL FIELD

The present invention relates to a stable crystal of (4R,5S,6S)-6-[(1R)-1-hydroxyethyl]-4-methyl-3-({4-[(5S)-5-methyl-2, 5-dihydro-1H-pyrrol-3-yl]-1,3-thiazol-2-yllthio)-7-oxo-l-azabicyclo[3.2.0]hept-2-ene-2-carboxylic acid (hereinafter this compound is abbreviated as Compound A) represented by the following formula 1: 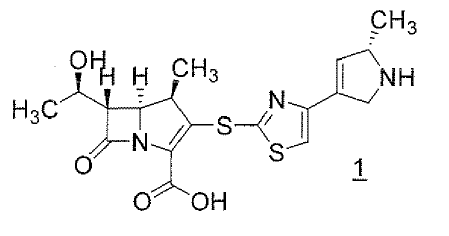 , a process for preparing the crystal and a pharmaceutical composition comprising the crystal.

### BACKGROUND OF ART

In a patent gazette (Patent document 1) related to the invention by the present inventors, it is reported that Compound A shows excellent activity against Gram positive bacteria, especially methicillin resistant staphylococcus and methicillin resistant coagulase negative staphylococcus.

However, although Compound A itself and a process for preparation of Compound A are described in the patent gazette, any crystal forms of Compound A is not concretely mentioned therein.
Patent document 1: WO 02/38564 gazette

### DISCLOSURE OF INVENTION

### Problem to be solved by invention

When a compound is used for a medicine, chemical and physical stability of the compound as an active pharmaceutical ingredient (API) as well as a pharmaceutical composition comprising the compound is required in order to maintain its quality and/or to make easy preserve the medicine.

The problem to be solved in the present invention is to provide Compound A having preferable properties for API, namely a stable crystal of Compound A.

### Means for solving the problem

The present inventors had the problem to obtain a stable Compound A to be usable for a medicine and extensively studied on Compound A. As a result they found that Compound A has crystalline polymorph and a crystal having a specific crystal form of Compound A is extremely stable, and then the invention was completed.

The outline on studies conducted by the inventors during that the invention was reached to completion are described below.

It was not easy to obtain a stable crystal of Compound A which was usable as API, as mentioned below.

When they tried a recrystalization method in order to desolvate an acetone solvated crystal which was first obtained, a non solvated crystal was obtained, but the crystal was rather unstable Form II crystal (See Reference example 2.). It was found that Form II crystal easily absorbs moisture in air and is partially transformed into Form III crystal. Therefore, Form II crystal was hardly preserved as a pure unitary crystal form and it was considered that the crystal is not suitable for API which is required to be unitary and stably preserved from the viewpoint of a crystal.

Next, it was tried to make Form II crystal absorb moisture to prepare hydrate crystal (See Reference example 3.). As a result, there was obtained trihydrate crystal (Form III crystal). However, the crystal was very unstable (See the result of stability test of Test 3.).

Furthermore, it was tried to desolvate acetone from an acetone solvate by washing an acetone solvated crystal of Compound A with an aquaous organic solvent containing an organic solvent which does not solvate with Compound A, and thereby there could be also obtained Form II crystal (See Reference example 4.).

As such, although various extensive studies were tried, there was not obtained a crystal of Compound A which was stable and did not contain an organic solvent as its solvate.

However, as described in Example 2, by washing a solvated crystal of Compound A with an aqueous organic solvent containing an organic solvent which did not solvate with Compound A and doing said procedure under heating, it was unexpectedly found that Form I crystal which did not contain an organic solvent as a solvate could be obtained and that Form I crystal was extremely stable under usual preservation conditions.

As β-lactam compounds have usually a tendency to be easily hydrolyzed, it is normal to avoid heating it at the present of water as a common knowledge in the art. Nevertheless, the present inventors could unexpectedly obtain stable Form 1 crystal by treating it under heating.

Furthermore, the speed of degradation of Compound A is accelerated in the presence of much more water as well as many other β-lactam compounds (See Comparative test 1.). Therefore, if Compound A was exposed under the circumstances of a moist gas, it was expected that the decomposition should be accelerated and such a process was not preferable for obtaining a stable crystal. Despite of such a practical expectation, the present inventors found that when a solvated crystal of Compound A was exposed for relatively long term under the circumstances of a moist gas (See a method described in Example 4.) and so on, stable Form I crystal was unexpectedly obtainable with a little bit degradation.

In the methods for preparing Form I crystal which was invented by the present inventors, the crystal is prepared via a solvated crystal of Compound A such as an acetone solvated crystal (Form IV crystal), a 2-prapanol solvated crystal (Form V crystal) and so on. Namely these solvated crystals are important key-intermediates for preparing useful Form I crystal.

As mentioned above, the present inventors completed the present invention.

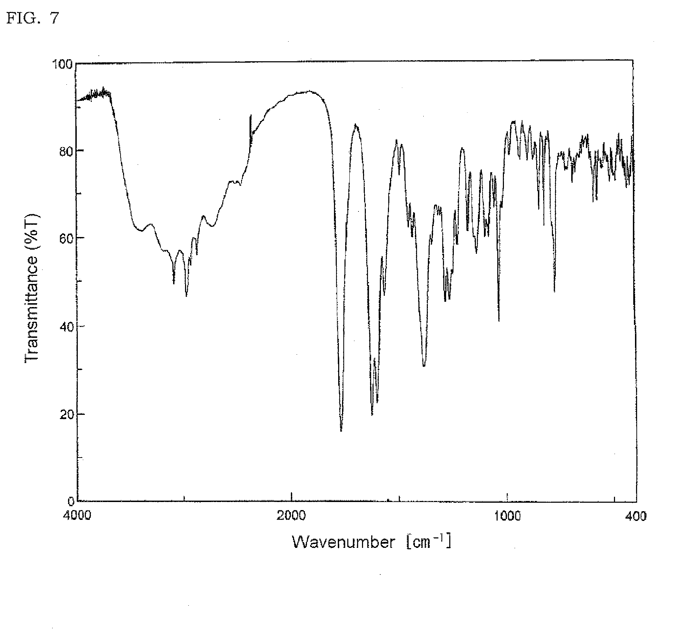

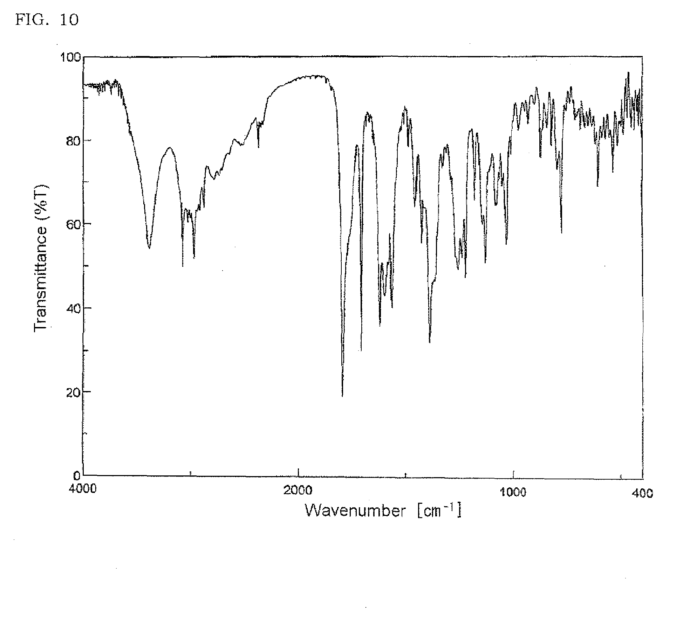

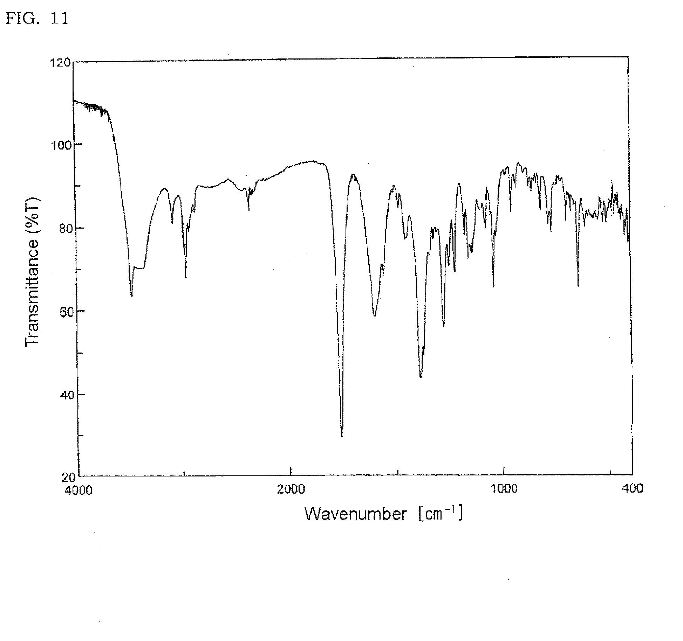

According to the present invention, the following aspects of the invention are provided.
[1] A crystal of (4R,5S,6S)-6-[(1R)-1-hydroxyethyl]-4-methyl-3-({4-[(5S)-5-methyl-2,5-dihydro-1H-pyrrol-3-yl]-1,3-thiazol-2-yl}thio)-7-oxo-1-azabicyclo[3.2.0]hept-2-ene-2-carboxylic acid (Compound A) which has powder X-ray diffraction pattern comprising 7.5±0.2, 9.9±0.2, 13.0±0.2, 14.5±0.2, 16.4±0.2 and 20.2±0.2 as diffraction peaks at angle of diffraction 2θ(°) measured by powder X-ray diffraction, which crystal is called Form 1 crystal.
[2] A crystal of Compound A which has powder X-ray diffraction pattern comprising 7.5±0.2, 9.9±0.2, 13.0±0.2, 14.5±0.2, 16.4±0.2 and 20.2±0.2 as diffraction peaks at angle of diffraction 2θ(°) measured by powder X-ray diffraction, and shows IR spectra of Figure 7 (Form I crystal).
[3] A process for preparing Form I crystal described in above item [1] which is characterized by desolvating a solvated organic solvent by a method selected from the group consisting of (a) a method for washing solvated crystal of Compound A with an aqueous organic solvent containing an organic solvent which does not solvate with Compound A, (b) a method for exposing solvated crystal of Compound A under the circumstance of a moist gas, and (c) a method for blowing a moist gas to solvated crystal of Compound A.
[4] The process for preparing Form I crystal according to above item [3] wherein the method for desolvation is a method for washing solvated crystal of Compound A with an aqueous organic solvent containing an organic solvent which does not solvate with Compound A.
[5] The process for preparing Form I crystal according to above item [4] wherein the washing with the aqueous organic solvent is carried out at 27-60°C.
[6] The process for preparing Form I crystal according to above item [3] wherein the method for desolvation is a method for exposing solvated crystal of Compound A under the circumstance of a moist gas, or a method for blowing a moist gas to solvated crystal of Compound A.
[7] The process for preparing Form 1 crystal according to above item [6] wherein the method for exposing solvated crystal of Compound A under the circumstance of a moist gas, or the method for blowing a moist gas to solvated crystal of Compound A is carried out at 15-40°C under 50-98%RH.
[8] The process for preparing Form I crystal described in above item [1] according to any one of above items [3] to [7] wherein the solvated crystal is acetone solvated crystal (Form IV crystal) or 2-propanol solvated crystal (Form V crystal).
[9] A process for preparing Form I crystal described in above item [1] which is characterized by desolvation by a method described in above item [3]; after obtaining solvated crystal of Compound A by following method (1) or (2):
   Method (1) for dissolving Compound A in a mixture of water and a water-miscible organic solvent which solvates with Compound A, followed by cooling the solution or
   Method (2) for dissolving Compound A in water, or a mixture of water and a water-miscible organic solvent which solvates with Compound A, followed by diluting the solution with a water-miscible organic solvent which solvates with Compound A.
[10] A process for preparing Form I crystal described in above item [1] which is characterized by desolvating a solvated organic solvent by a method selected from the group consisting of (a) a method for washing solvated crystal of Compound A with an aqueous organic solvent containing an organic solvent which does not solvate with Compound A, (b) a method for exposing solvated crystal of Compound A under the circumstance of a moist gas, and (c) a method for blowing a moist gas to solvated crystal of Compound A; after obtaining purified solvated crystal of Compound A by following method (3) or (4):
   Method (3) for dissolving solvated crystal of Compound A in a mixture of water and a water-miscible organic solvent which solvates with Compound A, followed by cooling the solution or
   Method (4) for dissolving solvated crystal of Compound A in water, or a mixture of water and a water-miscible organic solvent which solvates with Compound A, followed by diluting the solution with a water-miscible organic solvent which solvates with Compound A.
[11] The process for preparing Form I crystal according to above item [9] or [10] wherein a. solubilizer is used when dissolving Compound A or solvated crystal of Compound A in water, or a mixture of water and a water-miscible organic solvent which solvates with Compound A.
[12] The process for preparing Form I crystal according to any one of above items [9] to [11] wherein the organic solvent which solvates with Compound A is acetone or 2-propanol
[13] The process for preparing Form I crystal according to any one of above items [3] to [5], and [8] to [12] wherein the organic solvent which does not solvate with Compound A is 2-butanone.
[14] A pharmaceutical composition comprising Form I crystal claimed in above item [1].
[15] The pharmaceutical composition according to above item [14] wherein the composition is an injectable composition.
[16] An acetone solvated crystal of Compound A which has powder X-ray diffraction pattern comprising 9.1±0.2, 11.9±0.2, 13.2±0.2, 19.8±0.2 and 20.9±0.2 as diffraction peaks at angle of diffraction 2θ(°) measured by powder X-ray diffraction (Form IV crystal).
[17] An acetone solvated crystal of Compound A which has powder X-ray diffraction pattern comprising 9.1±0.2, 11.9±0.2, 13.2±0.2, 19.8±0.2 and 20.9±0.2 as diffraction peaks at angle of diffraction 2θ(°) measured by powder X-ray diffraction and shows IR spectra of Figure 10 (Form IV crystal).
[18] A 2-propanol solvated crystal of Compound A which has powder X-ray diffraction pattern comprising 7.9±0.2, 8.4±0.2, 17.2±0.2, 19.5±0.2, and 26.2±0.2 as diffraction peaks at angle of diffraction 2θ(°) measured by powder X-ray diffraction (Form V crystal).
[19] A 2-propanol solvated crystal of Compound A which has powder X-ray diffraction pattern comprising 7.9±0.2, 8.4±0.2, 17.2±0.2, 19.5±0.2, and 26.2±0.2 as diffraction peaks at angle of diffraction 26(°) measured by powder X-ray diffraction and shows IR spectra of Figure 11 (Form V crystal).

### Effects of Invention

As the crystal of Compound A of the present invention (Form I crystal) has a specific crystal form as mentioned above, the crystal has superiority in physical or chemical stability and therefore, it has merits to maintain for long term the quality of Compound A.

### BRIEF DESCRIPTION OF DRAWINGS

Fig. 1 shows powder X-ray diffraction pattern on Form I crystal. Horizontal axis shows angle of diffraction 2θ(°) and vertical axis shows intensity (cps) (Hereinafter the axes of Fig. 2 to 5 show the same meanings).
Fig. 2 shows powder X-ray diffraction pattern on Form II crystal.
Fig. 3 shows powder X-ray diffraction pattern on Form III crystal.
Fig. 4 shows powder X-rxay diffraction pattern on Form IV crystal.
Fig. 5 shows powder X-ray diffraction pattern on Form V crystal.
Fig. 6 shows powder X-ray diffraction patterns on Form I to V crystals by arranging on the same horizontal axis. The diffraction patterns respectively show Form I, Form II, Form III, Form IV and Form V crystals beginning at the top.
Fig. 7 shows IR spectra on Form I crystal. Horizontal axis shows wave number (cm⁻¹) and vertical axis shows transparece (%). Hereinafter the axes of Fig. 8 to 11 show the same meanings.
Fig. 8 shows IR spectra on Form II crystal.
Fig. 9 shows IR spectra on Form III crystal.
Fig. 10 shows IR spectra on Form IV crystal.
Fig. 11 shows IR spectra on Form V crystal.

### DESCRIPTION OF EMBODIMENTS

The present invention is explained in more detail below.

The crystal of Compound A of the present invention is Form I crystal. "Form I crystal of Compound A" means the crystal of Compound A which has powder X-ray diffraction pattern comprising 7.5, 9.9, 13.0, 14.5, 16.4 and 20.2 as diffraction peaks at angle of diffraction 2θ(°) measured by powder X-ray diffraction.

Further, values of diffraction peaks at angle of diffraction 2θ(°) may have a little bit measurement deviations according to an instrument for measurement, a condition for measurement and so on. Therefore, concretely said deviation error may be allowable within ±0.2, preferably ±0.1.

Form I crystal of Compound A is non-solvated crystal and amount of its residual organic solvent is usually 1.0% w/w or less, more preferably 0.5% w/w or less. Furthermore, amount of water containing therein is usually 2% w/w or less, more preferably 1.5% w/w or less.

Form I crystal of Compound A is more stable comparing with amorphous Compound A and other polymorphs of Compound A such as Form II crystal and Form III crystal. Namely Form I crystal is extremely preferable from the viewpoint of stability in case of using it as API.

A solvated crystal of Compound A which is a starting material of Form I crystal of Compound A is prepared by following methods.

A solvated crystal of Compound A can be prepared by dissolving amorphous Compound A in a mixture of water and a water-miscible organic solvent which solvates with Compound A, followed by cooling the solution; or by dissolving amorphous Compound A in water or a mixture of water and a water-miscible organic solvent which solvates with Compound A, followed by diluting it with a water-miscible organic solvent which solvates with Compound A. Furthermore, an aqueous crude product containing Compound A obtained by a chemical synthesis can be also used as a starting material without isolating. Furthermore, by adding seed crystals to the reaction mixture, crystallization can be accelerated.

The above water-miscible organic solvent which solvates with Compound A includes for example, acetone, ethanol, 2-propanol, tert-butanol, tetrahydrofuran, 1,4-dioxane, acetonitrile, dimethoxyethane and so on, or a mixture thereof, preferably acetone, 2-propanol, tert-butanol or tetrahydrofuran, and more preferably acetone or 2-propanol.

The solvated crystal can be purified by dissolving the solvated crystal thus obtained in a mixture of water and a water-miscible organic solvent which solvates with Compound A, followed by cooling it; or by dissolving in water or a mixture of water and a water-miscible organic solvent which solvates with Compound A, followed by diluting it with a water-miscible organic solvent which solvates with Compound A. In case of dissolving the solvated crystal, a solubilizer may be used if necessary, such as carbonate salts like potassium carbonate or sodium carbonate, hydrogencarbonates like potassium hydrogencarbonate or sodium hydrogencarbonate, aliphatic carboxylates like sodium acetate, sodium propionate or sodium octanoate, or a mixture thereof, preferably hydrogencarbonates or aliphatic carboxylates, and more preferably sodium hydrogencarbonate or sodium octanoate. Furthermore, in case of use of a solubilizer agent, neutralization may be carried out on the course of process. Examples of the neutralizing agent are methanesulfonic acid, phosphoric acid, octanoic acid, hydrochloric acid, sulfuric acid and so on. The neutralizing agent may be used by diluting with water or an organic solvent, if necessary.

Removal of the insoluble matters and sterilization are possible by filtering the solution with a filter after dissolving Compound A. When the solution is sterilized by filtration with a filter and all equipments which are used in the process are previously sterilized, it is possible to obtain said solvated crystal in aseptic condition by sterilizing all the solutions added later with filters.

The solvated crystal of Compound A prepared above is usually obtained as a solvated crystal which consists of one molecular of Compound A and about one molecular of organic solvent.

For example, when an organic solvent which is solvated is acetone or 2-propanol, content of acetone or 2-prapanal is usually 9 to 15% w/w, preferably 10 to 14% w/w, more preferably 11 to 13% w/w.

Thus obtained solvated crystal can be transformed into other solvated crystal by washing it with an aqueous organic solvent containing an organic solvent which is different from the solvated solvent and which solvates with Compound A.

An acetone solvated crystal of Compound A is Form IV crystal. "Form IV crystal of Compound A" means a crystal of Compound A which has powder X-ray diffraction pattern comprising 9.1, 11.9, 13.2, 19.8 and 20.9 as diffraction peaks at angle of diffraction 2θ(°) measured by powder X-ray diffraction.

Furthermore, a 2-propanol solvated crystal of Compound A is Form V crystal. "Form V crystal of Compound A" means a crystal of Compound A which has powder X-ray diffraction pattern comprising 7.9,8.4, 17.2, 19.5 and 26.2 as diffraction peaks at angle of diffraction 2θ(°) measured by powder X-ray diffraction.

Further, values of diffraction peaks at angle of diffraction 2θ(°) may have a little bit measurement deviations according to an instrument for measurement, a condition for measurement and so on. Therefore, concretely said deviation error may be allowable within ±0.2, preferably ±0.1.

Form I crystal of Compound A is prepared by following methods.

Form I crystal of Compound A is obtained by washing a solvated crystal of Compound A with an aqueous organic solvent which contain an organic solvent which does not solvate with Compound A, to desolvate the organic solvent which solvates with it.

The organic solvent, which does not solvate with Compound A includes, for example 2-butanone.

The aqueous organic solvents may further include an organic solvent which solvates with Compound A such as 2-propanol as well as water and an organic solvent which does not solvate with Compound A.

Amount of the aqueous organic solvent is not limited, but it is usually amount enough to get good slurry-like fluidization. Content of an organic solvent in the aqueous organic solvent is for example, 45% to 95% w/w, preferably 50 to 90% w/w, more preferably 55% to 80% w/w when an organic solvent which does not solvate with Compound A and an organic solvent which solvates with Compound A are combined. When an organic solvent which solvates with Compound A is used, ratio of the organic solvent which solvates with Compound A to total amount of organic solvents is for example, 60% w/w or less, preferably 50% w/w or less, more preferably 20% w/w or less.

Washing is usually carried at 27-60°C, preferably 30-55°C, more preferably 35-50°C. Especially when a solvated crystal of Compound A is an acetone solvated crystal, the preferable washing temperature is 30 to 55°C, more preferably 35 to 50°C.

Washing of a solvated crystal of Compound A is preferably conducted under stirring. Addition of a solvent for washing or warming is not specially limited, but the following methods are illustrated:
Method for mixing a previously prepared aqueous organic solvent and a solvated crystal of Compound A, followed by warming to said washing temperature;
Method for warming a previously prepared aqueous organic solvent to said washing temperature, followed by adding the aqueous organic solvent to a solvated crystal of Compound A; or
Method for mixing only an organic solvent with a solvated crystal of Compound A, followed by warming it in slurry state to said washing temperature, and followed by dropping water or an aqueous organic solvent.

When washing a solvated crystal of Compound A after purifying the crystal, it may be carried out in a filter without taking out a purified solvated crystal from it. In case that a solvated crystal of Compound A is aseptically obtained, Form I crystal can be obtainable still aseptically without taking out the solvated crystal from the filter by washing the solvated crystal with an aqueous organic solvent previously sterilized by filtration with a filter.

On the other hand, Form 1 crystal of Compound A can be also obtainable by desolvating an organic solvent which solvates, more specifically either by exposing solvated crystal of Compound A under the circumstance of a moist gas or by blowing a moist gas to a solvated crystal of Compound A. In this case, the moist gas is usually at 15-40°C and 50-98%RH , preferably at 15-30°C and 75-95%RH . The gas for preparing the moist gas is not limited as long as it does not react with Compound A in a desolvating process, but usually air or nitrogen gas is used, preferably nitrogen gas from the viewpoint of prevention of accidents.

In case of exposing solvated crystal of Compound A under the circumstances of a moist gas, or blowing a moist gas to solvated crystal of Compound A, the procedure is carried out under continuously or intermittently stirring, if necessary.

When this procedure is carried out after purifying a solvated crystal of Compound A, it may be carried out in a filter without taking out purified solvated crystal from the filter. In case that solvated crystal of Compound A is aseptically obtained, Form I crystal can be obtainable still aseptically without taking out the solvated crystal from the filter by exposing to or blowing the moist gas previously sterilized by filtration with a filter.

Form I crystal of Compound A of the present invention which can be prepared starting from an acetone solvated crystal or a 2-propanol solvated crystal of Compound A has following features.

As acetone and 2-propanol are relatively cheap, the solvated crystal can be prepared with lower cost.

Acetone and 2-propanol belong to class 3 regulated by ICH (International Conference on Harmonization of Technical Requirements for Registration of Pharmaceuticals for Human Use) and even if said solvents are remained in the final product, problems on safety are relatively a little.

An acetone solvated crystal (Form IV crystal) and a 2-propanol solvated crystal (Form V crystal) are stable themselves (See result of stability test of Test example 3.).

Therefore, it is the most preferable mode for preparing Form I crystal to prepare it starting from an acetone solvated crystal or a 2-propanol solvated crystal of Compound A by above mentioned methods.

The crystal of the present invention (Form I crystal of Compound A) itself or a pharmaceutical composition prepared by mixing it with a pharmaceutically acceptable carrier can be used, for example as an antibacterial agent for many kinds of mammalians including human.

The pharmaceutical composition of the present invention includes, for examples oral formulations such as tablets, capsules, pills, granules, powders, solutions, syrups or emulsions; pharmaceutical compositions for injection such as freeze dried preparation, powder-filled formulation, aquaous solutions for injection; external formulations such as ointments, creams, lotions or patches; aerosols; suppositories. These formulations are prepared with a pharmaceutically acceptable carrier in accordance with conventional methods.

The pharmaceutically acceptable carriers include various organic or inorganic carriers which are conventionally used as materials for pharmaceutical formulations, such as excipients, lubricants, binders or disintegrants in solid formulations; excipients, buffers, solubilizing agents or stabilizers in freeze dry formulations and powder-filled formulations; solvents, solubilizing agents, suspending agents, tonicity agents, buffers or soothing agents in solutions (an aqueous solution for injection). If necessary, other additives such as preservative agents, antioxidants, coloring agents or sweetening agents can be used.

Among the pharmaceutical compositions of the present invention, the composition for injection is preferable. In case of application of the composition for injection, it can be applied after the composition is dissolved in distilled water or a saline solution. Conventional sterilized solubilizing agents may be used.

It is essential that pharmaceutical formulations for intravenous, intraarterial, percutaneous, intramuscular, or intraperitoneal injections are aseptically manufactured. The pharmaceutical formulations for injection include freeze-drying formulations, powder-filled formulations and aqueous solutions for injection.

Among them sterilization can be carried out by filtration of a solution before being freeze-dried in case of freeze-drying formulations, or in the step of an aqueous solution in case of aqueous solutions for injection.

However, in case of powder-filled formulations, as API itself or a mixture of API with additives is filled into a vial, it is difficult to sterile finally it in the course for preparing the formulation. Therefore, it is necessary to prepare aseptic API in order to prepare aseptic powder-filled formulations.

It is possible to prepare an aseptic crystal of the present invention (Form I crystal of Compound A) via following steps (1) and (2).
(1) In a step of preparation of a solvated crystal of Compound A, a solution of Compound A is sterilized by filtration with a filter.
(2) All steps after the above step (1) including a step of desolvation of solvated crystal of Compound A are aseptically carried out.

Thus obtained aseptic Form I crystal of Compound A is extremely splendid as API of powder-filled preparation for injection. The present invention includes aseptic Form I crystal of Compound A, which is prepared by above methods, and also aseptic powder-filled formulations for injection comprising Form I crystal of Compound A.

The present invention is explained more in detail by illustrating Examples, Reference examples, Tests and Reference tests, but the present invention is not limited by them.

### EXAMPLES

### Reference example 1

### Preparation for amorphous Compound A

Amorphous Compound A was prepared in accordance with a method described in example 1 of WO 02/38564.

### Example 1

### Preparation of an acetone solvated crystal of Compound A (Form IV crystal)

Amorphous Compound A prepared by Reference example 1 was dissolved in water (100mg/ml). Thereto was added acetone ten times as much as water and resulting white suspension was stimulated by a spatula and supersonic to obtain seed crystals.

Then amorphous Compound A (1.0g) prepared by Reference example 1 was dissolved in a mixture of water (10ml) and acetone (10ml), and to the solution were added the above seed crystals at about 20°C to crystallize. After standing it at 0°C for 1 hour, acetone 10ml was added thereto, followed by standing it for 30 minutes. The resulting crystals were collected, washed and dried to give an acetone solvated crystal of Compound A (Form IV crystal) (870mg). Its acetone content was 10.6% w/w).

### Reference example 2

### Preparation of Form II crystal of Compound A

An acetone solvated crystal of Compound A (Form IV crystal) (0.30g) prepared above was dissolved in water (3.1g). After quickly filtering, the filtrate was taken in a flask. The filtrate was stirred at room temperature at about 25°C to crystallize. Separately an acetone solvated crystal of Compound A (Form IV crystal) (0.70g) was dissolved in water (7.1g). After quickly filtering the filtrate was transferred to the flask which contained the crystal. After the flask was cooled in an ice bath and then kept at the same temperature, the crystal was collected by filtration, washed and dried to give the crystal (0.65g). Part of the crystal was again dried in vacuum for 24 hours and dried nitrogen gas was introduced to relief the vacuum condition avoiding the absorbtion of the moisture, to give Form II crystal of Compound A which was identified by IR and XRD. Its water content was 0.4% w/w.

### Reference example 3

### Preparation of Form III crystal of Compound A

Part of Form II crystal of Compound A prepared by Reference example 2 was allowed to stand under about 84%RH at 23°C for 28 hours to give Form III crystal of Compound A which was identified by IR and XRD. Its water content was 11.8% w/w.

### Reference example 4

### Preparation of Form II crystal of Compound A

To an acetone solvated crystal of Compound A (Form IV crystal) (4.0g) was added a mixture of water; 2-propanol; and 2-butanone (4:2:11) (60g). After the mixture was stirred for about 40 minutes at 24 to 25°C, the crystal was collected by filtration, washed with the above mixed solvent and 2-butanone and dried to give Form II crystal of Compound A which was identified by IR and XRD. Its water, acetone, 2-propanol and 2-butanone contents were 0.8% w/w, less than 0.05% w/w, less than 0.05% w/w and 0.18% w/w respectively.

### Reference example 5

### Preparation of Form III crystal of Compound A

To an acetone solvated crystal of Compound A (Form IV crystal) (5.0g) was added a mixture of water; 2-propanol; and 2-butanone (4:2:11) (75g). After the mixture was stirred for about 70 minutes at 24 to 25°C, the crystal was collected by filtration, washed with the above mixed solvent and 2-butanone and dried. Thus obtained crystal was allowed to stand under about 82%RH at 25°C for 7 0.5 hours to give Form III crystal of Compound A (4.60g) which was identified by IR and XRD. Its water, acetone, 2-propanol and 2-butanone contents were 12.05% w/w, less than 0.05% w/w, less than 0.05% w/w and 0.17% w/w respectively.

### Example 2

### Preparation of Form I crystal of Compound A

(1) An acetone solvated crystal of Compound A (Form IV crystal) (9.7g) was dissolved in an aqueous solution (162g) containing sodium octanoate (8.3% w/w) cooled at 5°C. After filtration in order to eliminate insoluble matters, to the filtrate was added 2-propanol (89g) and the solution was neutralized with methanesulfonic acid (18% w/w) to around pH 6.5. After adding acetone (533g) at room temperature (about 25°C) and being kept stirring, the solution was gradually cooled to 5°C. The resulting crystals were collected by filtration, washed and dried to give a purified acetone solvated crystal of Compound A (Form IV crystal) (8.8g).
(2) To an acetone solvated crystal of Compound A (Form IV crystal) (8.3g) prepared by step (1) was added a mixture of 2-propanol and 2-butanone (1:4) (27g). After elevating to 40°C, thereto was added water (18g). The resulting crystals were collected by filtration, washed and dried to give Form I crystal of Compound A (6.6g) which was identified by IR and XRD. Its water, 2-propanol, 2-butanone and acetone contents were 0.7% w/w, 0.06% w/w, 0.16% w/w and less than 0.05% w/w respectively.

### Example 3

### Preparation of Form I crystal of Compound A

To an acetone solvated crystal of Compound A (Form IV crystal) (2.0g) was added a mixture of water; 2-propanol and 2-butanone (4:2:11) (30g). After stirring at room temperature (about 25°C) for 20 minutes, the solution was stirred for 15 minutes at 45°C. The resulting crystals were collected by filtration, washed with above mixed solvent and 2-butanone and dried to give Form I crystal of Compound A (1.7g) which was identified by IR and XRD. Its water, 2-propanol, 2-butanone and acetone contents were 0.52% w/w, less than 0.05% w/w, 0.19% w/w and less than 0.05% w/w respectively.

### Example 4

### Preparation of Form I crystal of Compound A

(1) 2-Propanol (24g) was added to an aqueous solution (46g) containing sodium hydrogencarbonate (2.5% w/w). Thereto was added an acetone solvated crystal or Compound A (Form IV crystal) (2.4g) to dissolve it at about 20°C. To the solution was added charcoal (0.02g), and the mixture was stirred and filtered in order to eliminate insoluble matters. After cooled to 0-5°C, the solution was neutralized with methanesulfonic acid to around pH 6.6. After adding acetone (71g), the solution was kept at 0 to 5°C. The resulting crystals were collected by filtration, washed and dried to give a purified acetone solvated crystal of Compound A (Form IV crystal) (2.3g).
(2) To an acetone solvated crystal of Compound A (Form IV crystal) (1.9g) prepared by step (1) was blowed at room temperature (about 25°C) nitrogen gas which was adjusted to about 89%RH for 8 hours, and then the crystal was dried to give Form I crystal of Compound A (1.6g) which was identified by IR. Its water and acetone contents were 1.5% w/w and less than 0.05% w/w respectively.

### Example 5

### Preparation of a 2-propanol solvated crystal of Compound A (Form V crystal)

2-Propanol (209g) was added to an aqueous solution (797g) containing sodium hydrogencarbonate (2.6% w/w). Thereto was added an acetone solvated crystal of Compound A (Form IV crystal) (49g) to dissolve it at about 20°C. To the solution was added 2-propanol (209g), charcoal (0.42g) and water (4g), and the mixture was stirred and filtered in order to eliminate insoluble matters. The solution was neutralized with an aqueous solution of methanesulfonic acid (67% w/w) to around pH 6.0. After adding 2-Propanol (3351g), seed crystals were seeded, and kept stirring. The resulting crystals were collected by filtration, washed and dried to give a 2-propanol solvated crystal of Compound A (Form V crystal) (44g) which was identified by IR. Its 2-propanol content was 12.55% w/w.

### Example 6

### Preparation of a 2-propanol solvated crystal of Compound A (Form V crystal)

2-Propanol (1.7g) was added to an aqueous solution (8.7g) containing sodium hydrogencarbonate (3.3% w/w). Thereto was added an acetone solvated crystal of Compound A (Form IV crystal) (49g) under ice cooling to dissolve it. To the solution was added charcoal (0.01g), and the mixture was stirred and filtered in order to eliminate insoluble matters. The solution was neutralized at about 1.7°C with octanoic acid at around pH 6.2. After seed crystals were seeded, 2-propanol was added thereto and kept stirring. The resulting crystals were collected by filtration, washed and dried to give a 2-propanol solvated crystal of Compound A (Form V crystal) (0.55g) which was identified by IR and XRD. Its water content was 0.5% w/w. Its content of Compound A was 85.8% w/w.

### Example 7

### Preparation of Form I crystal of Compound A

To a 2-propanol solvated crystal of Compound A (Form V crystal) (3.0g) was added a mixture of water; 2-propanol and 2-butanone (4:1:4) (15g). After the solution was stirred for 60 minutes at 27°C, the resulting crystals were collected by filtration, washed and dried to give Form I crystal of Compound A (2.4g) which was identified by IR. Its water, 2-propanol and 2-butanone were 1.1% w/w, 0.05% w/w, and 0.14% w/w respectively.

### Example 8

### Preparation of Form I crystal of Compound A

To a 2-propanol solvated crystal of Compound A (Form V crystal) (0.23g) was blowed at about 30°C nitrogen gas which was adjusted to about 75%RH for 2 hours, and then the crystal was dried to give Form I crystal of Compound A which was identified by IR. Its propanol content was 0.05% w/w.

### Example 9

### Preparation of a 2-propanol solvated crystal of Compound A (Form V crystal)

To an acetone solvated crystal of Compound A (Form IV crystal) (2.0g) was added a mixture of water and 2-propanol (1:1) (20g). The solution was stirred for 30 minutes at 28°C, and the resulting crystals were collected by filtration, washed with above mixed solvent and 2-propanol, and dried to give a 2-propanol solvated crystal of Compound A (Form V crystal) (1.8g) which was identified by IR and XRD. Its water and 2-propanol contents were 0.3% w/w and 12.1% w/w respectively.

### Example 10

### Preparation of Form IV crystal of Compound A

An acetone solvated crystal of Compound A (Form IV crystal) (5.5g) was dissolved in an aqueous solution (51g) containing sodium octanoate (18% w/w) cooled at 5°C. After filtration in order to eliminate insoluble matters, to the filtrate was added 2-prapanal (29g) and the solution was neutralized with an aqueous solution of methanesulfonic acid (18% w/w) to around pH 6.5. After adding acetone (170g) at 10°C and being kept stirring, the solution was gradually cooled to 0°C. The resulting crystals were collected by filtration, washed and dried to give a purified acetone solvated crystal of Compound A (Form IV crystal) (5.2g).

### Example 11

### Preparation of Form I crystal of Compound A

To an acetone solvated crystal of Compound A (Form IV crystal) (4.0g) was added a mixture of 2-propanol and 2-butanone (1:4) (20g). After the temperature was elevated at 45°C, thereto was added water (5g) and the solution was kept stirring. The resulting crystals were collected by filtration, washed and dried to give Form 1 crystal of Compound A (3.45g) which was identified by IR. Its water, 2-propanol, 2-butanone and acetone contents were 1.9% w/w, 0.07% w/w, 0.16% w/w and less than 0.05% w/w respectively.

### Tests

According to following analytical tests, physical values on each crystal were obtained. As indexing their values, stability tests on each crystal were conducted.

### Analytical test

### Test 1

### Powder X-ray diffraction analysis (XRD test)

XRD was measured on each crystal prepared by examples and Reference examples.

The test sample was filled on a silicon-nonreflecting plate and diffraction patterns on the test sample was measured with XRD instrument (RAD-RB RU-200, manufacture by Rigaku) under following conditions:
Source of X-ray: Cu-Ka radiation, voltage in tube: 50kV, electric current in tube: 150mA, scanning speed: 4°per minute, step width: 0.02°, diffraction angle: at 2-40°.

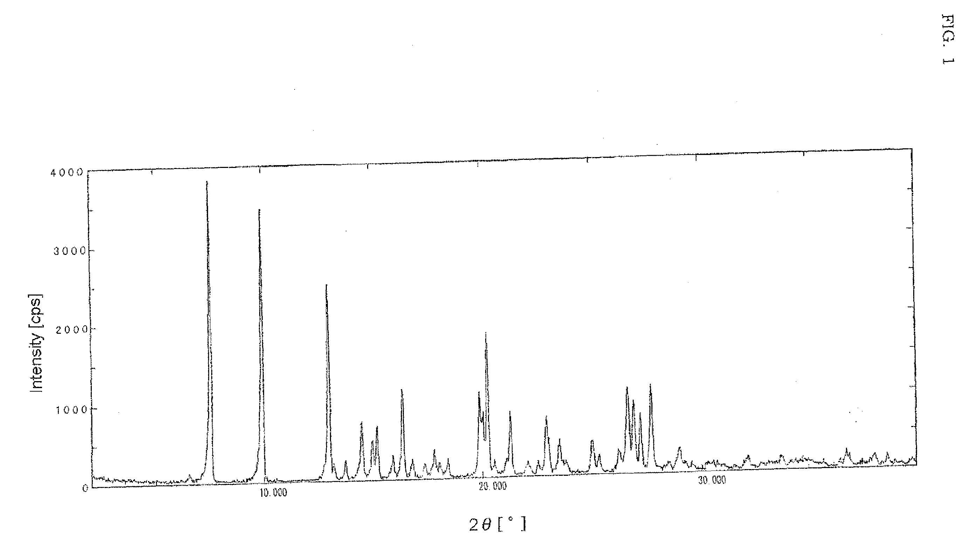

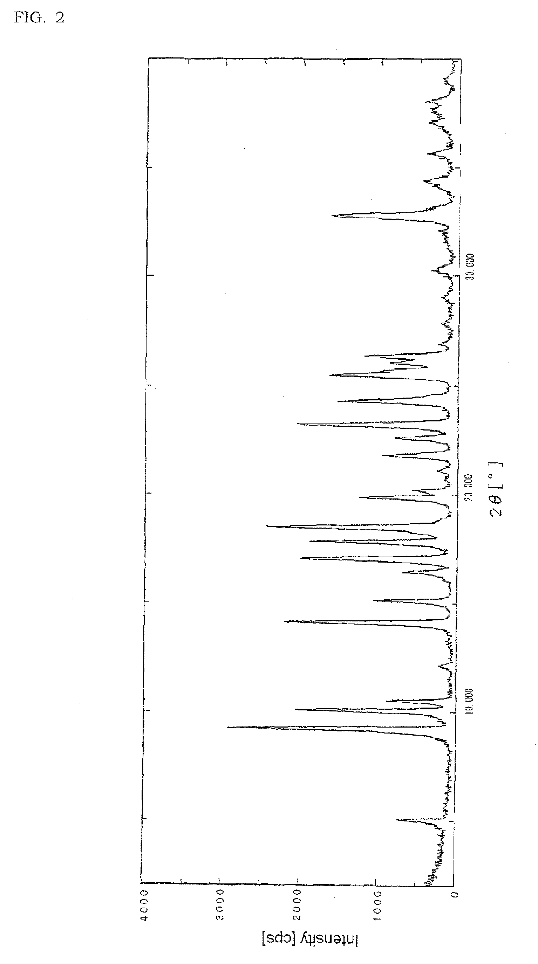

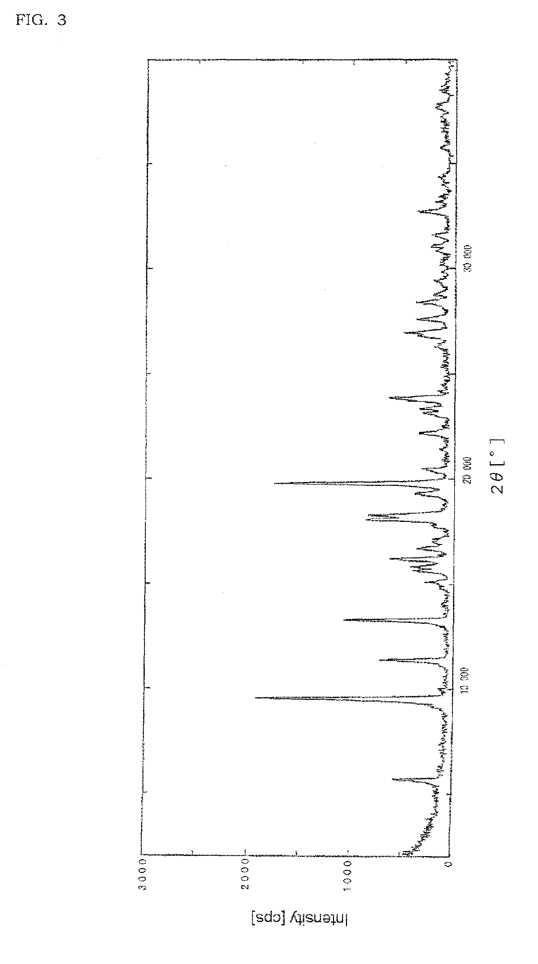

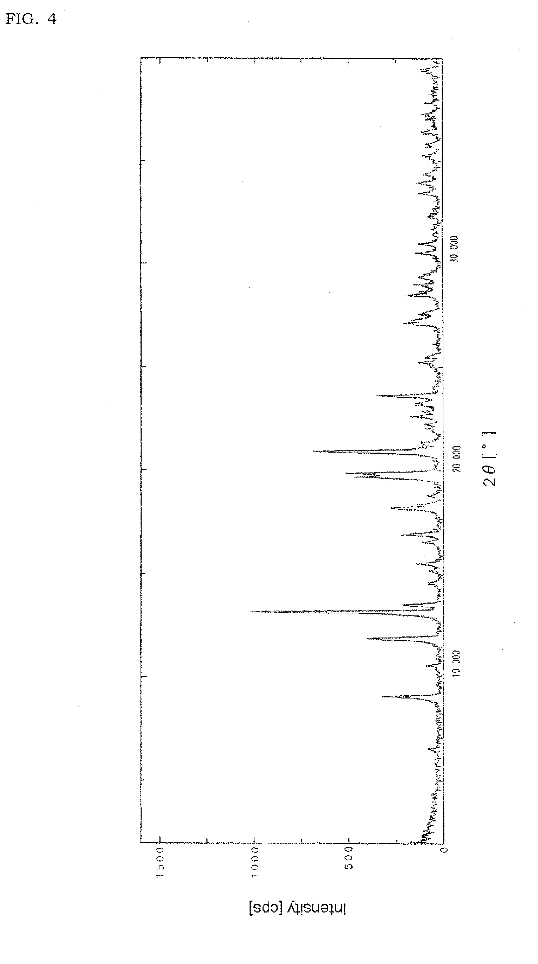

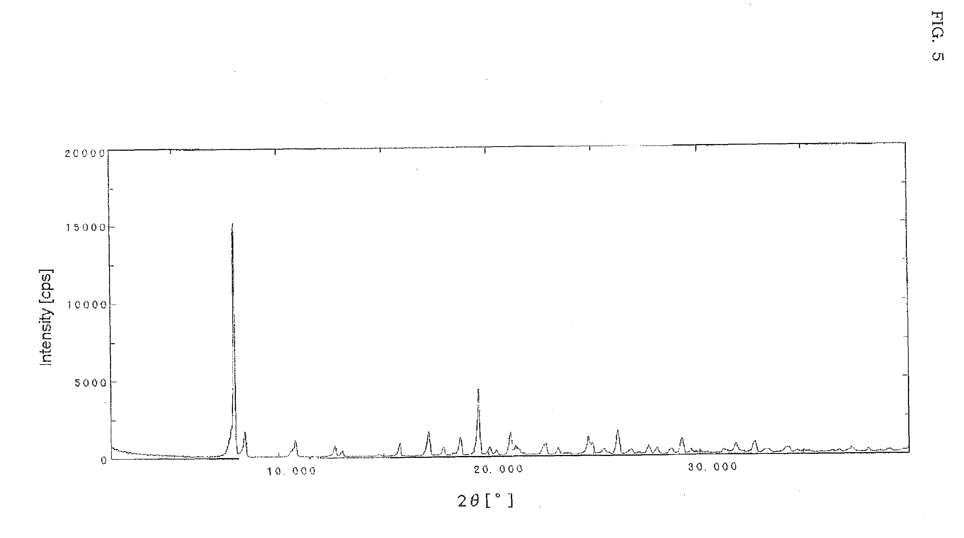

Obtained diffraction patterns (XRD spectra) are shown in Fig. 1 to Fig. 6.

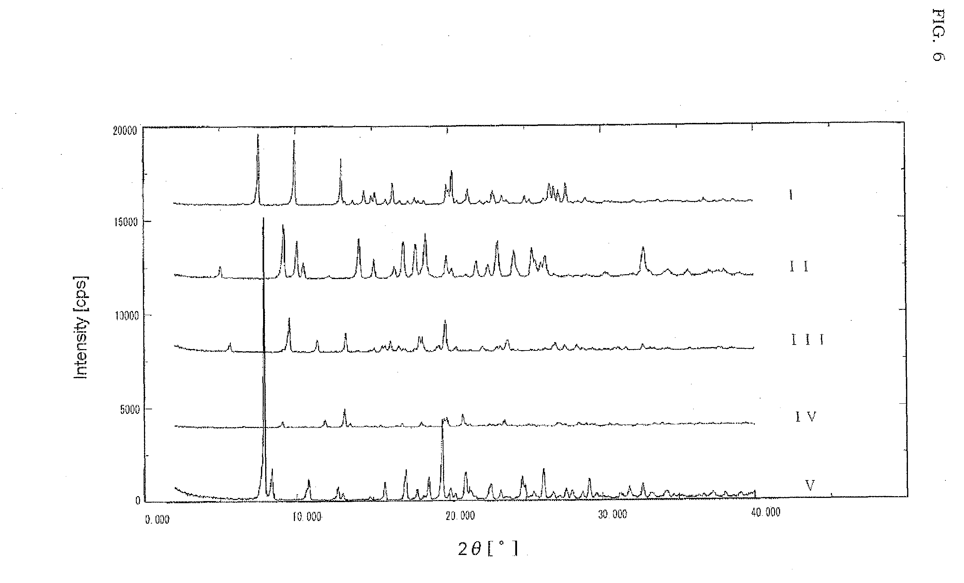

As shown in Fig. 6, it was observed that X-ray diffraction patterns on each samples were different, and crystals represented and prepared by Examples and Reference examples were different each other from these data, and showed characteristic diffraction patterns by XRD test.

Therefore, these crystals in the present specification are named Form I crystal, Form 11 crystal, Form III crystal, Form IV crystal and Form V crystal, respectively according to these powder X-ray diffraction patterns.

In case of identification (specification) of crystal forms, the crystal forms are judged by characteristic diffraction peaks based on X-ray diffraction pattern shown in Fig. 1 to 6.

Principal diffraction patterns and characteristic diffraction patterns specified from Fig. 1 to Fig. 6 are shown below.

Further values of diffraction peaks at angle of diffraction 2θ(°) may have a little bit measurement deviations according to an instrument for measurement, a condition for measurement and so on. Therefore, concretely said deviation error may be allowable within ±0.2, preferably ±0.1.

### [Form I crystal]

Principal diffraction peak: 2θ(°) = 7.5, 9.9, 13.0, 14.5, 15.9, 16.4, 19.9, 20.2, 26. 6, 26.9, 27.2, 27.7

Characteristic diffraction peak: 2θ(°) = 7.5, 9.9, 13.0, 14.5, 16.4, 20.2

### [Form II crystal]

Principal diffraction peak: 2θ(°) = 9.2, 10.1, 14.1, 17.0, 17.8, 18.5, 19.9, 23.2, 24.3, 25.5, 26.4, 32.8

Characteristic diffraction peak: 2θ(°) = 9.2, 10.1, 14.1, 17.0, 17.8, 18.5, 23.2

### [Form III crystal]

Principal diffraction peak: 2θ(°) = 5.6, 9.5, 11.4, 13.3, 16.2, 18.0, 18.3, 19.8, 23.8

Characteristic diffraction peak: 2θ(°) = 9.5, 13.3, 18.0, 19.8, 23.8

### [Form IV crystal]

Principal diffraction peak: 2θ(°) = 9.1, 11.9, 13.2, 16.9, 18.2, 18.3, 19.7, 19.8, 20.9, 23.6

Characteristic diffraction peak: 2θ(°) = 9.1, 11.9, 13.2, 19.8, 20.9

### [Form V crystal]

Principal diffraction peak: 2θ(°) = 7.9, 8.4, 10.8, 15.8, 17.2, 18.7, 19.5, 21.1, 24.8, 26.2, 29.2

Characteristic diffraction peak: 2θ(°) = 7.9, 8.4, 17.2, 19.5, 26.2

### Test 2

### Infrared spectra (IR spectra)

The test sample was well mixed with potassium bromide to make a tablet, and the sample was measured by using IR spectrophotometer (FT/IR-4200, manufactured by JASCO) under following conditions:
Range of measurement: 4000-400cm⁻¹, integrated frequency 50 times, resolution power: 2cm⁻¹

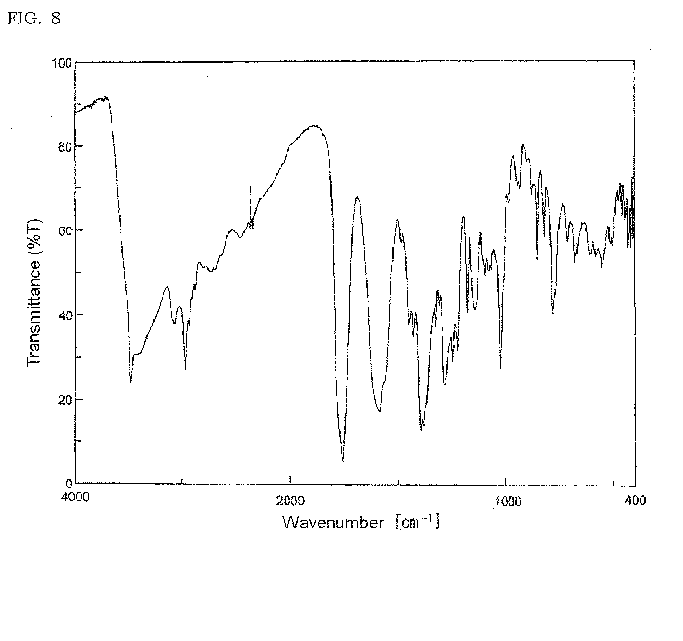

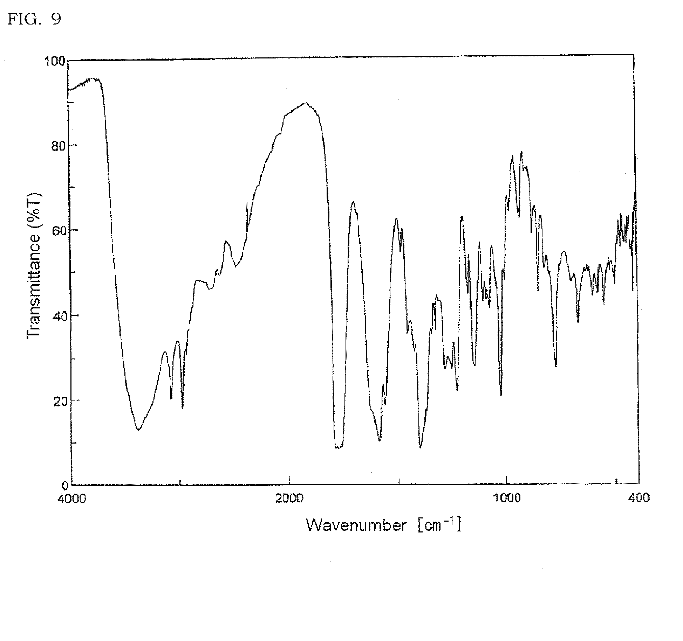

The obtained spectra are shown in Fig. 7 to 11. Fig. 7: Form I crystal, Fig. 8: Form II crystal, Fig. 9: Form III crystal, Fig. 10: Form IV crystal, and Fig. 11: Form V crystal.

### Test 3

### Stability test

Stability test on each test sample was conducted under following storage conditions. Results on amorphous Compound A, Form I crystal, Form II crystal, Form III crystal, Form IV crystal and Form V crystal are shown in Tables 1, 2, 3, 4, 5 and 6, respectively.

Evaluation was based on change of area percentage of Compound A with HPLC (High Performance Liquid Chromatography) under following conditions:

### HPLC:

Column: SUMIPAX ODS-A212 (silica gel binding with octadecyl group, particle size 5µm; 6.0mm ϕ x 15cm),
mobile phase A solution: a mixture of 0.005mol/L phosphate buffer (pH7.0) and acetonitrile (300:23(v/v)),
mobile phase B solution: acetonitrile,
solvent for sample: water and acetonitrile (90:10(v/v)),
wavelength: 220nm,
column temperature: constant temperature around 25°C,
flow rate: l.OmL/min,
injection amount: 10µL, and
gradient condition:

| Time (min.) | Mobile phase A (%) | Mobile phase B (%) |
|---|---|---|
| 0.00 | 100.0 | 0.0 |
| 30.00 | 100.0 | 0.0 |
| 60.00 | 64.0 | 36.0 |
| 70.00 | 22.0 | 78.0 |
| 70.10 | 100.0 | 0.0 |
| 90.00 | Finished | |

### Preparation of test sample: Compound A (5mg)/solvent (10mL)

**Table 1**

| Result of stability on amorphous Compound A in a closed vial at 50°C (HPLC surface area %) | |
|---|---|
| Preservation term | Compound A |
| Initial | 97.95% |
| 1 day | 91.68% |
| 1 week | 73.40% |
| 3 weeks | 54.53% |
| 6 weeks | 39.93% |
| 8 weeks | 41.33% |

**Table 2**

| Result of stability on Form I crystal in a closed vial at 55°C (HPLC surface area %) | | |
|---|---|---|
| Preservation term | Compound A | Difference from initial value |
| Initial | 99.77% | 0% |
| 2 weeks | 99.60% | -0.17% |
| 4 weeks | 99.60% | -0.17% |

**Table 3**

| Result of stability on Form II crystal in a closed vial at 55°C (HPLC surface area %) | | |
|---|---|---|
| Preservation term | Compound A | Difference from initial value |
| Initial | 99.72% | 0% |
| 2 weeks | 98.42% | -1.30% |
| 4 weeks | 97.56% | -2.16% |

**Table 4**

| Result of stability on Form III crystal in a closed vial at 55°C (HPLC surface area %) | |
|---|---|
| Preservation term | Compound A |
| Initial | 99-66% |
| 2 weeks | 9.68% |
| 4 weeks | 7.78% |

**Table 5**

| Result of stability on Form IV crystal in a closed vial at 55°C (HPLC surface area %) | |
|---|---|
| Preservation term | Compound A |
| Initial | 99.81% |
| 2 weeks | 99.66% |
| 4 weeks | 99.70% |

**Table 6**

| Result of stability on Form V crystal in a closed vial at 55°C (HPLC surface area %) | |
|---|---|
| Preservation term | Compound A |
| Initial | 99.91% |
| 2 weeks | 99.72% |
| 4 weeks | 99.73% |

As being clear from results on the above stability tests, it was found that Form I crystal was extremely stable under normal storage condition although Form II crystal was relatively unstable, and Form III crystal was unstable. Furthermore, it was found that Form II crystal easily absorbed moisture under high humidity to be gradually crystal transformed into Form III crystal. On the contrary Form III crystal easily released hydrated water under dried condition (under lower humidity or reduced pressure) to be gradually transformed into

### Form II crystal.

Therefore, it is extremely difficult to keep a pure and unitary crystal form on Form II crystal or Form III crystal because Form II crystal or Form III crystal absorbs water or releases hydrated water under usual storage condition to easily produce a mixture of them. As it is desirable that API is maintained in a pure and stable crystal form, neither Form II crystal nor Form III crystal is preferable API from this viewpoint.

On the other hand, it was found that Form IV crystal and Form V crystal are stable under usual storagecondition from the above results. Therefore, their crystals are superior as intermediates for preparation of Form I crystal. However, as they contain an organic solvent as a solvate, they can not be said to be desirable for API.

From above results, it was found that Form I crystal is preferable for use as API.

### Test 4

### Stability test on Form I crystal under humidity condition

In order to confirm the stability on Form I crystal under humidity condition was conducted following test. The test was carried out by using moisture-absorbed Form I crystal, which was prepared by storing Form I crystal obtained by the method of Example 2 under the atmosphere with 53%RH in a whole day in order to make it absorb moisture.

**Table 7**

| Result of stability of Form I crystal at 60°C (HPLC surface area %) | |
|---|---|
| Preservation term | Moisture-absorbed Form I crystal |
| Initial water content | 1.5% |
| Initial | 99.0% |
| 2 weeks | 98.4% |
| 4 weeks | 98.3% |

### Comparative test 1

### Stability test on Form III crystal under humidity condition

To confirm of stability on Form II crystal under humidity condition was conducted following test.

The stability was tested by using Form II crystal prepared by method of Reference example 2 and by using moisture-absorbed Form II crystal (making absorb moisture by blowing for 30 minutes a nitrogen gas adjusted to 42 to 44% relative humidity).

**Table 8**

| Result of stability of Form II crystal at 54°C (HPLC surface area %) | | |
|---|---|---|
| Preservation term | Form II crystal | Moistlare-absorbed Form II crystal |
| Initial water content | 1.35% | 5.46% |
| Initial | 99.30% | 99.44% |
| 14 hours | 98.92% | 96.61% |
| 103 hours | 98.18% | 79.84% |

From the above results, it was found that Form II crystal easily absorbs moisture under usual handling condition and therefore its stability is greatly decreased. On the other hand, it was found that Form I absorbs a little moisture under usual handling condition and its stability is excellent.

### INDUSTRIAL APPLICABILITY

According to the present invention, it can provide a stable crystal of Compound A which has a preferable property as API and a pharmaceutical composition comprising a stable crystal of Compound A.

## Claims

1. A crystal of (4R,5S,6S)-6-[(1R)-1-hydroxyethyl]-4-methyl-3-({4-[(5S)-5-methyl-2,5-dihydro-1H-pyrrol-3-yl]-1,3-thiazol-2-yl}thio)-7-oxo-1-azabicyclo[3.2.0]hept-2-ene-2-carbaxylic acid (hereinafter this compound is abbreviated as Compound A) which has powder X-ray diffraction pattern comprising 7.5±0.2, 9.9±0.2, 13.0±0.2, 14.5±0.2, 16.4±0.2 and 0.2±0.2 as diffraction peaks at angle of diffraction 2θ(°) measured by powder X-ray diffraction, which crystal is called Form I crystal.

2. A crystal of Compound A which has powder X-ray diffraction pattern comprising 7.5±0.2, 9.9±0.2, 13.0±0.2, 14.5±0.2, 16.4±0.2 and 0.2±0.2 as diffraction peaks at angle of diffraction 2θ(°) measured by powder X-ray diffraction, and shows IR spectra of Fig. 7 (Form I crystal).

3. A process for preparing Form I crystal described in claim 1 which is **characterized by** desolvating a solvated organic solvent by a method selected from the group consisting of (a) a method for washing solvated crystal of Compound A with an aqueous organic solvent containing an organic solvent which does not solvate with Compound A, (b) a method for exposing solvated crystal of Compound A under the circumstance of a moist gas, and (c) a method for blowing a moist gas to solvated crystal of Compound A.

4. The process for preparing Form I crystal according to claim 3 wherein the method for desolvation is a method for washing solvated crystal of Compound A with an aqueous organic solvent containing an organic solvent which does not solvate with Compound A.

5. The process for preparing Form I crystal according to claim 4 wherein the washing with the aqueous organic solvent is carried out at 27-60°C.

6. The process for preparing Form I crystal according to claim 3 wherein the method for desolvation is a method for exposing solvated crystal of Compound A under the circumstance of a moist gas, or a method for blowing a moist gas to solvated crystal of Compound A.

7. The process for preparing Form I crystal according to claim 6 wherein the method for exposing solvated crystal of Compound A under the circumstance of a moist gas, or a method for blowing a moist gas to solvated crystal of Compound A is carried out at 15-40°C under 50-98%pH.

8. The process for preparing Form I crystal described in claim 1 according to any one of claims 3 to 7 wherein the solvated crystal is acetone solvated crystal (Form IV crystal) or 2-propanol solvated crystal (Form V crystal).

9. A process for preparing Form I crystal described in claim 1 which is **characterized by** desolvating a solvated organic solvent by a method selected from the group consisting of (a) a method for washing solvated crystal of Compound A with an aqueous organic solvent containing an organic solvent which does not solvate with Compound A, (b) a method for exposing solvated crystal of Compound A under the circumstance of a moist gas, and (c) a method for blowing a moist gas to solvated crystal of Compound A;
after obtaining solvated crystal of Compound A by following method (1) or (2):
Method (1) for dissolving Compound A in a mixture of water and a water-miscible organic solvent which solvates with Compound A, followed by cooling the solution or
Method (2) for dissolving Compound A in water, or a mixture of water and a water-miscible organic solvent which solvates with Compound A, followed by diluting the solution with a water-miscible organic solvent which solvates with Compound A.

10. A process for preparing Form I crystal described in claim 1 which is **characterized by** desolvating a solvated organic solvent by a method selected from the group consisting of (a) a method for washing solvated crystal of Compound A with an aqueous organic solvent containing an organic solvent which does not solvate with Compound A, (b) a method for exposing solvated crystal of Compound A under the circumstance of a moist gas, and (c) a method for blowing a moist gas to solvated crystal of Compound A;
after obtaining purified solvated crystal of Compound A by following method (3) or (4):
Method (3) for dissolving solvated crystal of Compound A in a mixture of water and a water-miscible organic solvent which solvates with Compound A, followed by cooling the solution or
Method (4) for dissolving solvated crystal of Compound A in water, or a mixture of water and a water-miscible organic solvent which solvates with Compound A,
followed by diluting the solution with a water-miscible organic solvent which solvates with Compound A.

11. The process for preparing Form I crystal according to claim 9 or 10 wherein a solubilizer is used when dissolving Compound A or solvated crystal of Compound A in water, or a mixture of water and a water-miscible organic solvent which solvates with Compound A.

12. The process for preparing Form I crystal according to any one of claims 9 to 11 wherein the organic solvent which solvates with Compound A is acetone or 2-propanol.

13. The process for preparing Form I crystal according to any one of claims 3 to 5, and 8 to 12 wherein the organic solvent which does not solvate with Compound A is 2-butanone.

14. A pharmaceutical composition comprising Form I crystal claimed in claim 1.

15. The pharmaceutical composition according to claim 14 wherein the composition is an injectable composition.

16. An acetone solvated crystal of Compound A which has powder X-ray diffraction pattern comprising 9.1±0.2, 11.9±0.2, 13.2±0.2, 19.8±0.2 and 20.9±0.2 as diffraction peaks at angle of diffraction 2θ(°) measured by powder X-ray diffraction (Form IV crystal).

17. A 2-propanol solvated crystal of Compound A which has powder X-ray diffraction pattern comprising 7.9±0.2, 8.4±0.2, 17.2±0.2, 19.5±0.2, and 26.2±0.2 as diffraction peaks at angle of diffraction 2θ(°) measured by powder X-ray diffraction (Form V crystal).
